# EUROPÄISCHE PATENTSCHRIFT

(11) **EP 2 103 942 B1**
(45) Veröffentlichungstag und Bekanntmachung des Hinweises auf die Patenterteilung: **25.07.2012**
(21) Anmeldenummer: 08005272.3
(22) Anmeldetag: 20.03.2008
(51) Int. Cl.: G01N 33/68

(54) **Verfahren zur Bestimmung des Typs einer entzündlich-rheumatischen Erkrankung in Synovialflüssigkeit**
Method for determining the nature of an inflammatory-rheumatic illness in synovial fluid
Procédé de détermination d'un type d'infection rhumatismale dans des synovies

(43) Veröffentlichungstag der Anmeldung: 23.09.2009
(73) Patentinhaber: Universitätsklinikum Freiburg, 79106 Freiburg (DE)
(72) Erfinder: Melchers, Inga, 79227 Schallstadt (DE)
(74) Vertreter: Keller, Günter

(56) Entgegenhaltungen:
- WO-A-00/53174
- US-A1- 2003 143 238
- MASSA MARGHERITA ET AL: "Differential recognition of heat-shock protein dnaJ-derived epitopes by effector and treg cells leads to modulation of inflammation in juvenile idiopathic arthritis" ARTHRITIS & RHEUMATISM, Bd. 56, Nr. 5, Mai 2007 (2007-05), Seiten 1648-1657, XP009101814 ISSN: 0004-3591
- ALBANI S ET AL: "IMMUNE RESPONSES TO THE ESCHERICHIA COLI DNAJ HEAT SHOCK PROTEIN IN JUVENILE RHEUMATOID ARTHRITIS AND THEIR CORRELATION WITH DISEASE ACTIVITY" JOURNAL OF PEDIATRICS, MOSBY-YEAR BOOK, ST. LOUIS, MO, US, Bd. 124, Nr. 4, 1. Januar 1994 (1994-01-01), Seiten 561-565, XP009051918 ISSN: 0022-3476
- ALBANI S ET AL: "POSITIVE SELECTION IN AUTOIMMUNITY: ABNORMAL IMMUNE RESPONSES TO A BACTERIAL DNAJ ANTIGENIC DETERMINANT IN PATIENTS WITH EARLY RHEUMATOID ARTHRITIS" NATURE MEDICINE, NATURE PUBLISHING GROUP, NEW YORK, NY, US, Bd. 1, Nr. 5, 1. Mai 1995 (1995-05-01), Seiten 448-452, XP002033102 ISSN: 1078-8956
- KRZEWSKI KONRAD ET AL: "Characterization of the anti-DnaJ monoclonal antibodies and their use to compare immunological properties of DnaJ and its human homologue HDJ-1." CELL STRESS AND CHAPERONES, Bd. 8, Nr. 1, April 2003 (2003-04), Seiten 8-17, XP002486149 ISSN: 1355-8145
- KURZIK-DUMKE URSULA ET AL: "Overexpression of human homologs of the bacterial DnaJ chaperone in the synovial tissue of patients with rheumatoid arthritis" ARTHRITIS AND RHEUMATISM, Bd. 42, Nr. 2, Februar 1999 (1999-02), Seiten 210-220, XP009101815 ISSN: 0004-3591

### SEQUENCE LISTING

<110> Universitätsklinikum
<120> verfahren zur Bestimmung des Typs einer rheumatoiden Erkrankung
<130> Hdj2
<160> 2
<170> PatentIn version 3.5
<210> 1
   <211> 1538
   <212> DNA
   <213> Homo sapiens
<400> 1
   ggacttccgg cgggtgacgc gcccgggttc ggctacaaaa gaggacggct gcggcgcgcc      60
   gggcggaact ttccagaacg ctcggtgaga ggcggaggag cggtaactac cccggctgcg     120
   cacagctcgg cgctccttcc cgctccctca cacaccggcc tcagcccgca ccggcagtag     180
   aagatggtga aagaaacaac ttactacgat gttttggggg tcaaacccaa tgctactcag     240
   gaagaattga aaaaggctta taggaaactg gctttgaagt accatcctga taagaaccca     300
   aatgaaggag agaagtttaa acagatttct caagcttacg aagttctctc tgatgcaaag     360
   aaaagggaat tatatgacaa aggaggagaa caggcaatta aagagggtgg agcaggtggc     420
   ggttttggct cccccatgga catctttgat atgttttttg gaggaggagg aaggatgcag     480
   agagaaagga gaggtaaaaa tgttgtacat cagctctcag taaccctaga agacttatat     540
   aatggtgcaa caagaaaact ggctctgcaa aagaatgtga tttgtgacaa atgtgaaggt     600
   agaggaggta agaaaggagc agtagagtgc tgtcccaatt gccgaggtac tggaatgcaa     660
   ataagaattc atcagatagg acctggaatg gttcagcaaa ttcagtctgt gtgcatggag     720
   tgccagggcc atggggagcg gatcagtcct aaagatagat gtaaaagctg caacggaagg     780
   aagatagttc gagagaagaa aattttagaa gttcatattg acaaaggcat gaaagatggc     840
   cagaagataa cattccatgg tgaaggagac caagaaccag gactggagcc aggcgatatt     900
   atcattgtgt tagatcagaa ggaccatgct gtttttactc gacgaggaga agaccttttc     960
   atgtgtatgg acatacagct cgttgaagca ctgtgtggct tccagaagcc aatatctact    1020
   cttgacaacc gaaccatcgt catcacctct catccaggtc agattgtcaa gcatggagat    1080
   atcaagtgtg tactaaatga aggcatgcca atttatcgta gaccatatga aaagggtcgc    1140
   ctaatcatcg aatttaaggt aaactttcct gagaatggct ttctctctcc tgataaactg    1200
   tctttgctgg aaaaactcct acccgagagg aaggaagtgg aagagactga tgagatggac    1260
   caagtagaac tggtggactt tgatccaaat caggaaagac ggcgccacta caatggagaa    1320
   gcatatgagg atgatgaaca tcatcccaga ggtggtgttc agtgtcagac ctcttaatgg    1380
   gccagtgaat aacactcact gctggcattt aatgtgcagt agtgaatgag tgaaggactg    1440
   taatcataat atgctcacta cttgctcttg tttttgtttt aataaactat agtagtgttt    1500
   taaaaagtta aatgaagaat aaacgcaaat ataaaagc                            1538
<210> 2
   <211> 397
   <212> PRT
   <213> Homo sapiens
<400> 2
   Met Val Lys Glu Thr Thr Tyr Tyr Asp Val Leu Gly Val Lys Pro Asn
   1               5                   10                  15
   Ala Thr Gln Glu Glu Leu Lys Lys Ala Tyr Arg Lys Leu Ala Leu Lys
               20                  25                  30
   Tyr His Pro Asp Lys Asn Pro Asn Glu Gly Glu Lys Phe Lys Gln Ile
           35                  40                  45
   Ser Gln Ala Tyr Glu Val Leu Ser Asp Ala Lys Lys Arg Glu Leu Tyr
       50                  55                  60
   Asp Lys Gly Gly Glu Gln Ala Ile Lys Glu Gly Gly Ala Gly Gly Gly
   65                  70                  75                  80
   Phe Gly Ser Pro Met Asp Ile Phe Asp Met Phe Phe Gly Gly Gly Gly
                   85                  90                  95
   Arg Met Gln Arg Glu Arg Arg Gly Lys Asn Val Val His Gln Leu Ser
               100                 105                 110
   Val Thr Leu Glu Asp Leu Tyr Asn Gly Ala Thr Arg Lys Leu Ala Leu
           115                 120                 125
   Gln Lys Asn Val Ile Cys Asp Lys Cys Glu Gly Arg Gly Gly Lys Lys
       130                 135                 140
   Gly Ala Val Glu Cys Cys Pro Asn Cys Arg Gly Thr Gly Met Gln Ile
   145                 150                 155                 160
   Arg Ile His Gln Ile Gly Pro Gly Met Val Gln Gln Ile Gln Ser Val
                   165                 170                 175
   Cys Met Glu Cys Gln Gly His Gly Glu Arg Ile Ser Pro Lys Asp Arg
               180                 185                 190
   Cys Lys Ser Cys Asn Gly Arg Lys Ile Val Arg Glu Lys Lys Ile Leu
           195                 200                 205
   Glu Val His Ile Asp Lys Gly Met Lys Asp Gly Gln Lys Ile Thr Phe
       210                 215                 220
   His Gly Glu Gly Asp Gln Glu Pro Gly Leu Glu Pro Gly Asp Ile Ile
   225                 230                 235                 240
   Ile Val Leu Asp Gln Lys Asp His Ala Val Phe Thr Arg Arg Gly Glu
                   245                 250                 255
   Asp Leu Phe Met Cys Met Asp Ile Gln Leu Val Glu Ala Leu Cys Gly
               260                 265                 270
   Phe Gln Lys Pro Ile Ser Thr Leu Asp Asn Arg Thr Ile Val Ile Thr
           275                 280                 285
   Ser His Pro Gly Gln Ile Val Lys His Gly Asp Ile Lys Cys Val Leu
       290                 295                 300
   Asn Glu Gly Met Pro Ile Tyr Arg Arg Pro Tyr Glu Lys Gly Arg Leu
   305                 310                 315                 320
   Ile Ile Glu Phe Lys Val Asn Phe Pro Glu Asn Gly Phe Leu Ser Pro
                   325                 330                 335
   Asp Lys Leu Ser Leu Leu Glu Lys Leu Leu Pro Glu Arg Lys Glu Val
               340                 345                 350
   Glu Glu Thr Asp Glu Met Asp Gln Val Glu Leu Val Asp Phe Asp Pro
           355                 360                 365
   Asn Gln Glu Arg Arg Arg His Tyr Asn Gly Glu Ala Tyr Glu Asp Asp
       370                 375                 380
   Glu His His Pro Arg Gly Gly Val Gln Cys Gln Thr Ser
   385                 390                 395

## Beschreibung

Eine Arthritis ist eine Gelenkentzündung, die durch folgende Beschwerden erkannt werden kann: Schmerzen, Schwellung, Überwärmung, Bewegungseinschränkung, Gelenkerguss, Gelenkemphysem, manchmal Rötung. Bei chronischen Verläufen kann es auch zu Erosionen kommen, die das Gelenk verändern und zerstören können. Im Verlauf kann es zu Fehlstellungen, Muskelverkürzungen und Versteifungen kommen, die oft besonders die kleinen Gelenke der Füße und Hände betreffen.

Es gibt sehr viele unterschiedliche Formen der Arthritis, die man nach unterschiedlichen Kriterien einteilen kann. Für die Prognose und Therapie einer arthritischen Erkrankung ist es wesentlich, dass möglichst einfach, frühzeitig und zuverlässig diagnostiziert werden kann, um welche Art von Arthritis es sich handelt und was die Ursache dieser Arthritis ist, damit möglichst frühzeitig die wirksamste Therapie ausgewählt werden kann. Ungefähr die Hälfte der Patienten mit undifferenzierter Arthritis entwickelt innerhalb der nächsten 5 Jahre eine wesentliche Funktionseinschränkung der betroffenen Gelenke, während die anderen einen milden Verlauf zeigen, teilweise auch ohne Therapie. Eine Prognose des wahrscheinlichen weiteren Verlaufs der Erkrankung ist daher wünschenswert.

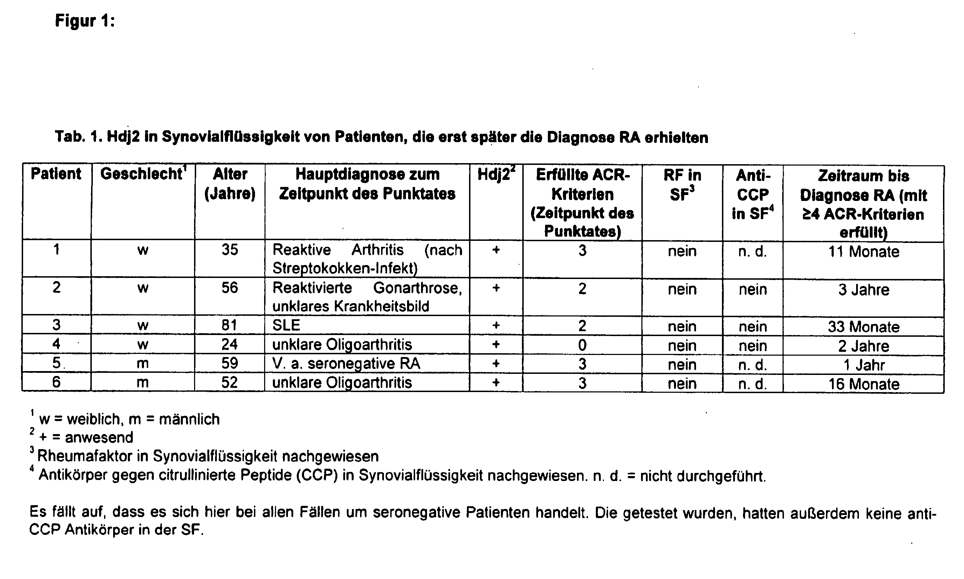

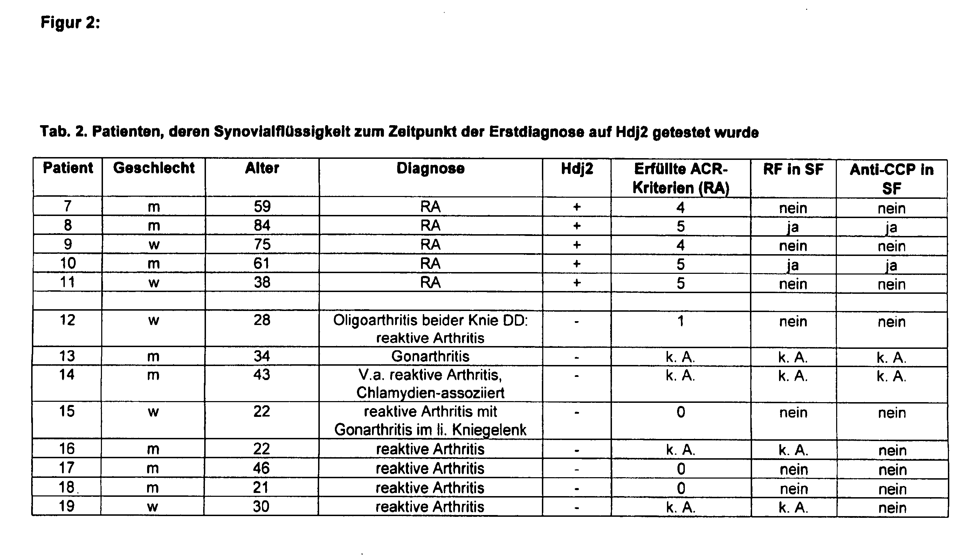

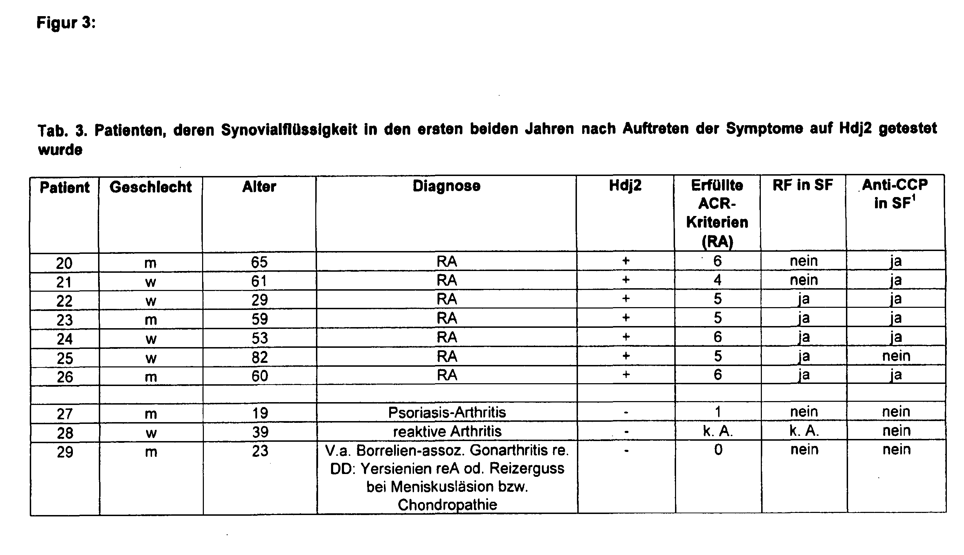

Massa et al., Arthritis & Rheumatism (2007), S. 1648-1657 messen die Immunantwort von humanen Zellen gegen das bakterielle DnaJ (Proliferationstest, Cytokin-Produktion), die Zellen stammen aus Patienten mit juveniler idiopathischer Arthritis (Fig. 2, 3). Einzelne Peptide des bakteriellen DnaJ werden mit homologen und nicht-homologen Peptiden der humanen J-Proteine HSJ1, HDJ1 und Hdj2 verglichen (Fig. 1, 4). Alle humanen Peptide ergaben keine signifikanten Unterschiede bei der Proliferation und der IFN-Y Synthese im Vergleich zu einem Kontrollpeptid (im Text erwähnt).

Albani et al., J of Pediatrics (1994), S. 561-565 untersuchen die Immunantwort gegen das bakterielle DnaJ, in Patienten mit juveniler rheumatoider Arthritis (Zusammenfassung). Von humanen J-Proteinen und ihrer Expression ist nicht die Rede.

Albani et al., Nature Medicine (1995), S. 448-452 untersuchen die Immunantwort gegen das bakterielle DnaJ, in Patienten mit rheumatoider Arthritis. Von humanen J-Proteinen und ihrer Expression ist nicht die Rede.

Die US 2003/0143238 bezieht sich auf arthritogene Peptide, die die Sequenz Q(K/R)RAA enthalten, das sogenannte "*shared epitope*", das sowohl im bakteriellen DnaJ als auch in einigen humanen HLA-DRB1-Molekülen vorkommt, die mit der rheumatoiden Arthritis assoziiert sind. HLA-DRB1-Moleküle sind an der Antigen-Präsentation beteiligt; sie sind keine J-Proteine. Das Hdj2 Molekül hat keine Q(K/R)RAA Sequenz, sondern an dieser Stelle KKREL, wobei man wissen muss, dass KR in dieser Position bei fast allen J-Proteinen vorkommt.

Krzewski et al., Cell Stress & Chaperones (2003), S. 8-17 beschreiben die Herstellung von monoklonalen Antikörpern gegen *E*. *coli* DnaJ in Mäusen. Sechs Antikörper werden genauer charakterisiert. Dabei werden DnaJ Peptide identifiziert, an die die Antikörper binden, und es wird getestet, ob sie an das humane J-Protein Hdj1 binden. Alle tun dies, ebenso ein polyklonales Kaninchenserum.

Die WO 00/53174 offenbart Verbindungen, die die Proteinexpression beeinflussen können. In dieser Patentanmeldung geht es um ein Verfahren zur Behandlung von Erkrankungen, bei denen Fehlfaltungen von Proteinen ein Problem darstellen. Soweit wir wissen, gehört die rheumatoide Arthritis nicht in diese Gruppe.

Kurzik-Dumke et al., Arthritis & Rheumatism (1999), S. 210-220 beschreiben in Synovialgewebe von RA Patienten ein für die RA spezifisches Molekül, dessen Identität ist jedoch bis heute unklar. Es könnte ein humanes J-Protein sein, bei dem es sich um ein Homolog des in *Drosophilia* beschriebenen Tid56 handeln könnte, da es ein ähnliches Molekulargewicht hat. Hdj2 wird in dieser Arbeit aber zumindest indirekt ausgeschlossen, da es i) kleiner ist und ii) von den verwendeten Antiseren weder im Immunoblot noch in der Immuncytochemie entdeckt werden kann.

In Lehr et al., ANN RHEUM DIS, 2008, col. 67 (Suppl. I), A29, 082 wird eine immunhistochemische Färbung in Immunoblots beschrieben. Dieser Arbeit kann aber nicht entnommen werden, dass Hdj2 in löslicher Form in der Synovialflüssigkeit vorkommt und, dass Hdj2 gerade bei Frühformen von entzündlichen rheumatischen Arthritiden als diagnostischer Marker geeignet sein könnte.

Schick et al., Immunobiology 1997, 197, 250 gehört zu einem Poster, das anlässlich eines European Workshop on Rheumatology Research gezeigt wurde. Beschrieben wurde in dieser Arbeit die Untersuchung von Synovialgewebe von lediglich 9 Patienten mit rheumatoider Arthritis. Mit einem Antikörper wurden Zellen im Gewebe, wir auch isolierte Zellen aus Synovialgewebe angefärbt. Auch in dieser Arbeit wird nicht offenbart, dass Hdj2 in löslicher Form in Synovialflüssigkeit vorkommt. Erst recht nicht offenbart oder gar nahegelegt wird, dass dieses Protein als diagnostischer Marker verwendet werden kann, um die rheumatoide Arthritis von anderen rheumatischen Erscheinungsformen zu unterscheiden.

Im Rahmen der vorliegenden Erfindung wurde herausgefunden, dass ein bestimmtes Protein mit der Bezeichnung Hdj2, das normalerweise nur intrazellulär vorkommt, in den Synovialflüssigkeiten von Patienten mit rheumatoider Arthritis (im folgenden RA) häufig, frühzeitig und in erheblichem Umfang auftritt, wodurch eine Differenzierung von Patienten mit rheumatoider Arthritis und anderen Patienten, die eine andere entzündlich-rheumatische Erkrankung, nämlich reaktive Arthritis oder Psoriasis-Arthritis, aufweisen, ermöglicht wird.

Hdj2, ein normalerweise intrazelluläres J-Protein, kommt als extrazelluläres Molekül in Synovialflüssigkeiten von Patienten mit rheumatoider Arthritis (RA) signifikant häufiger vor als in Synovialflüssigkeiten von Patienten mit anderen Arthritiden (nicht-RA Patienten). Es zeigt in der Synovialflüssigkeit signifikante Korrelationen mit dem Auftreten von zwei mit der RA assoziierten Autoantikörpern (Rheumafaktor, anti-CCP). Es korreliert in RA-Patienten mengenmäßig positiv mit dem Entzündungsgrad, gemessen als Anzahl infiltrierender Leukocyten. Hdj2 findet sich stark überexprimiert in der synovialen Deckzellschicht von Patienten mit RA, aber nicht in Synovialgewebe von Patienten mit Osteoarthrose.

Das J-Protein Hdj2 (DNAJA1, Hsj2, Hsdj, dj-2) ist eines von etwa 40 bekannten menschlichen J-Proteinen. J-Proteine zeichnen sich durch eine hochkonservierte J-Domäne aus, die eine hohe Homologie zu DnaJ von E.coli hat (man nennt sie daher auch DnaJ-ähnliche Proteine). J-Proteine gelten als Co-Chaperone der Mitglieder der HSP70 Familie, mit denen sie über die J-Domäne interagieren, und als Chaperonmaschine funktionieren. Die bereits bekannte Messenger-RNA ist als DNA-Sequenz im Sequenzprotokoll als SEQ ID NO:1 wiedergegeben.

Die Nucleinsäure kodiert für das Hdj2-Protein. Die bereits bekannte Aminosäuresequenz ist im Sequenzprotokoll als SEQ ID NO:2 wiedergegeben.

Im Rahmen der vorliegenden Erfindung wurde überraschend festgestellt, dass sich Hdj2 als ein früher Marker für die Diagnose der rheumatoiden Arthritis eignet, weil gerade in den frühen Stadien der Erkrankung ein erhöhter Gehalt an Hdj2 in der Synovialflüssigkeit von solchen Patienten aufgefunden wird, die später eine rheumatoide Arthritis entwickeln. Dies ist insofern überraschend, als vorhergehende Untersuchungen zeigten, dass Hdj2 auch in der Synovialflüssigkeit von Patienten aufgefunden wird, die verschiedene Erkrankungen des rheumatischen Formenkreises entwickeln. Im vorliegenden Fall besteht ein wesentlicher Aspekt der vorliegenden Erfindung darin, dass Hdj2 in frühen Erkrankungsstadien bei denjenigen Patienten in der Synovialflüssigkeit auftritt, die später eine rheumatoide Arthritis entwickeln. Bei Patienten, die andere Formen einer entzündlich-rheumatischen Erkrankung entwickeln, tritt dagegen dieser Marker in der Synovialflüssigkeit nicht oder nur in sehr geringem Ausmaß auf.

Entzündlich-rheumatische Erkrankungen beginnen häufig mit schmerzhaften Schwellungen der Gelenke. Bei der RA ist die sogenannte Morgensteifigkeit, mit einer Dauer von mehr als 1 Stunde, ein weiteres frühes Merkmal. Eine entzündliche Polyarthritis, bei der mehr als 3 Gelenkregionen betroffen sind, die die Hand- und Fingergelenke einschließt, und die symmetrisch ist, könnte eine beginnende RA sein. Die klinischen Zeichen einer beginnenden RA müssen mindestens 6 Wochen bestehen, um eine Diagnose stellen zu können. Autoantikörper, wie der Rheumafaktor und die anti-CCP-Antikörper, können im Blut nachweisbar sein. Diese gehen teilweise den Beschwerden voraus, können aber auch erst später auftreten. Erosionen und Rheumaknoten entwickeln sich hingegen im Allgemeinen erst im weiteren Verlauf der Erkrankung. Das Ausmaß der Schwellungen, der Morgensteifigkeit und der Schmerzen kann im Verlauf sehr schwanken. Eine undifferenzierte Arthritis ist noch ungeklärt und nicht klassifiziert. Wurde eine Diagnose gestellt, so kann man die ersten 2-3 Jahre noch von einer frühen (rheumatoiden) Arthritis sprechen. Patienten mit undifferenzierter Arthritis weisen oft nur 2-3 der ACR-Kriterien auf, und die klinischen Zeichen der Arthritis bestehen oft nur 4 Wochen lang. So ist es nicht möglich, die Patienten mittels der ACR-Kriterien als RA zu klassifizieren.

Unter einem frühen Stadium der Erkrankung wird im vorliegenden Fall die Zeitspanne verstanden, die mit dem Einsetzen rheumatischer Erkrankungen beginnt und sich über einen Zeitraum von bis zu zwei Jahren, besonders bevorzugt bis zu einem Jahr und ganz besonders bevorzugt bis zu sechs Monaten erstreckt. Unter dem Einsetzen einer entzündlich-rheumatischen Erkrankung wird das Auftreten der ersten Symptome verstanden. Zu den ersten Symptomen gehört eine Morgensteifigkeit für eine Dauer von mehr als einer Stunde und das Auftreten von Gelenkschwellungen. Im Verlauf einer entzündlich-rheumatischen Erkrankung tritt Arthritis in zwei oder mehr Gelenkregionen, insbesondere Arthritis an Hand- oder Fingergelenken auf. Rheumaknoten treten in der Regel erst viel später auf.

Ein weiteres Symptom für den Beginn einer entzündlich-rheumatischen Erkrankung kann sein, wenn Gelenkschwellungen in mehr als zwei Gelenken über einen Zeitraum von mehr als sechs Wochen bestehen. Der genaue Zeitpunkt des Auftretens einer entzündlich-rheumatischen Erkrankung kann nur sehr schwierig präzise bestimmt werden. Die ersten Symptome werden subjektiv von den Patienten festgestellt und ein exaktes Datum kann nur selten festgelegt werden. In diesen Fällen wird daher die Erstdiagnose einer entzündlich-rheumatischen Erkrankung als Zeitpunkt definiert, mit dem die frühe Phase der Erkrankung beginnt.

Hdj2 eignet sich als ein früher Marker für RA, da es vor der Stellung dieser Diagnose in Synovialgewebe (eine Patientin, s. hierzu Beispiel 1) und in Synovialflüssigkeit (mehrere Patienten, s. Tabelle 1) auffindbar war. Es eignet sich als Marker für eine Differentialdiagnose der RA, da es bei Personen mit fraglichen Diagnosen vorhanden war, die später auch oder statt dessen die Diagnose RA erhielten (Tabelle 1). Hdj2 wurde zum Zeitpunkt der Diagnosestellung in der Synovialflüssigkeit von Patienten mit RA gefunden, die durch die Erfüllung von ≥4 der 7 ACR-Kriterien sicher als RA klassifiziert werden konnten (Tabelle 2). Andererseits wurde Hdj2 zum Zeitpunkt der Diagnosestellung nicht in der Synovialflüssigkeit von Patienten gefunden, die nicht als RA klassifiziert wurden (Tabelle 2). Bei Synovialflüssigkeiten von Patienten mit früher Arthritis (höchstens 2 Jahre nach Erstdiagnose) verhielt es sich entsprechend, d.h. Hdj2 war bei Patienten mit RA vorhanden, bei Patienten mit einer anderen Diagnose aber nicht (entsprechende Daten sind in Tabelle 3 zusammengefasst).

Die rheumatoide Arthritis ist nicht nur eine Erkrankung mehrerer Gelenke, sondern sie betrifft den ganzen Körper. Bei der rheumatoiden Arthritis handelt es sich um die häufigste Form der Gelenkentzündung, die unbehandelt zur Zerstörung der Gelenke, zu frühzeitiger Arbeitsunfähigkeit und - da sie eine systemische Erkrankung ist und auch innere Organe betroffen sind, z.B. durch endotheliale Dysfunkion - zu erhöhter Morbidität führt.

Bei der rheumatoiden Arthritis kommt es in den Gelenken zu einer Entgleisung der normalen Entzündungsprozesse. Der eigentliche Auslöser dieser Erkrankung ist bisher noch nicht identifiziert, die rheumatoide Arthritis wird jedoch zu den Autoimmunerkrankungen gezählt. Die Pathogenese ist noch unverstanden. Man nimmt an, dass sowohl genetische wie auch Umweltfaktoren eine Rolle spielen, es könnte sich also um eine multifaktoriell bedingte Krankheit handeln. Da Verwandte eines Patienten in der Regel ein höheres Risiko tragen, ebenfalls an rheumatoider Arthritis zu erkranken, geht man davon aus, dass es genetische Ursachen geben könnte. Bis heute sind allerdings nur wenige Gene, bzw. Allele bekannt, die mit der rheumatoiden Arthritis assoziiert sind und eine größere Gruppe von Patienten betreffen.

Nach derzeitigem Kenntnisstand betrifft die für die rheumatoide Arthritis wichtigste genetische Assoziation den sehr polymorphen HLA-Komplex. Personen, die ein oder zwei HLA-DRB1 Allele tragen, welche ein Aminosäuremotiv, genannt "shared epitope", besitzen, haben ein höheres Risiko zu erkranken als Personen ohne dieses Motiv; jedoch gibt es auch viele Gesunde mit dem "shared epitope" (SE). Dieses Motiv besteht aus 5 Aminosäuren, und kann im Einbuchstabencode QKRAA, QRRAA oder RRRAA lauten. In der kaukasischen Bevölkerung Europas und Nordamerikas ist QKRAA das häufigste SE.

HLA-DRB1 Gene codieren HLA-DR Moleküle, die als MHC Klasse II Moleküle in der Immunantwort eine wichtige Rolle spielen. Diese Moleküle präsentieren auf den Oberflächen von antigenpräsentierenden Zellen Peptide, die von Antigenen (z. B. bei einer Infektion) oder auch von Autoantigenen (z. B. bei einer Autoimmunerkrankung) stammen können. Nachfolgend können T-Lymphocyten aktiviert werden, die ihrerseits B-Lymphocyten helfen, Antikörper gegen die Antigene oder Autoantigene zu synthetisieren. Das Auftreten von Autoantikörpern, die mit Autoantigenen reagieren, stellt ein Merkmal einer Autoimmunantwort dar. Auch die RA kann als Autoimmunerkrankung betrachtet werden, wegen der genetischen Assoziation mit HLA-DRB1 Allelen, und wegen des Auftretens von Autoantikörpern (dem Rheumafaktor, der humanes IgG erkennt, und seit kurzer Zeit einer weiteren Klasse von Autoantikörpern, die gegen citrullinierte Proteine gerichtet sind, und hier als anti-CCP zusammengefasst werden können).

Das "shared epitope" wurde nun interessanterweise nicht nur auf HLA-DR Molekülen gefunden, sondern die Sequenz QKRAA kommt auch bei vielen Bakterien vor. Als Prototyp hierfür gilt *E*. *coli* (Darmbakterien), und ihr DnaJ Protein. Dieses trägt eine J-Domäne, in der das Motiv QKRAA des SE liegt. Weiterhin wurde gezeigt, dass das SE in DnaJ wichtig für die Interaktion mit dem Partnermolekül von DnaJ ist, dem DnaK (*E*. *coli*). DnaK ist ein Hitzeschockprotein (HSP) der HSP70-Familie. HSP70 Proteine gibt es in allen Organismen, ebenso die mit ihnen interagierenden J-Proteine (genannt nach der J-Domäne des DnaJ). Die HSP70- und J-Proteine bilden zusammen eine Chaperonmaschine (chaperon = engl. Anstandsdame). Sie wirken also bei der korrekten Faltung von anderen Proteinen mit, wie es z. B. nach einem Hitzeschock einer Zelle notwendig wird, damit die Zelle überleben kann.

Beim Menschen gibt es ca.12 Gene für HSP70-Proteine und >40 Gene für J-Proteine. Von vielen ist nur die Nukleinsäure-Sequenz bekannt. Die entsprechend vielfältig zusammengesetzten HSP70/J-Protein-Chaperonmaschinen wirken intrazellulär an vielen Funktionen der Zellen mit, nicht nur in Stress-Situationen, sondern auch bei z. B. Transport oder Proteinsynthese. Auch an der Antigenpräsentation sind sie beteiligt. Das Motiv des "shared epitope" findet sich bei den bekannteren humanen J-Proteinen nicht.

Im Synovialgewebe von Patienten mit RA werden mindestens ein HSP70 Molekül (das Hsc70) und ein J-Protein (das Hdj2) sehr stark überexprimiert. Es ist bekannt, dass beide miteinander kooperieren, und dass Hsc70 an der Allel-spezifischen Antigenpräsentation bei DRB1*0401-positiven Personen wesentlich beteiligt ist.

Ohne an eine spezielle Theorie gebunden sein zu wollen, wird im Rahmen der vorliegenden Erfindung davon ausgegangen, dass eine Allel-spezifische Antigenpräsentation am Beginn der Erkrankung steht. Die daran beteiligten Moleküle, zu denen möglicherweise auch das Hdj2 gehört, können damit als Marker für das Entstehen und Auftreten einer rheumatoiden Arthritis verwendet werden. Dieser Marker kann eingesetzt werden für eine Subgruppierung der Patienten, als Marker für Differentialdiagnosen, Verlaufskontrollen, Prognosen und auch für die Überprüfung der Wirksamkeit der Therapie. Überraschend war die Feststellung, dass das Protein Hdj2, ein normalerweise intrazellulär auftretendes Protein, in der Synovialflüssigkeit extrazellulär auftritt und eine direkte Korrelation zur Klassifikation der entzündlich-rheumatischen Erkrankung ermöglicht. Besonders überraschend ist, dass dieses Auftreten schon in einem relativ frühen Stadium erfolgt, was eine frühzeitige Diagnose ermöglicht.

Bei Beginn einer Arthritis schwellen oft die Gelenke und diese werden dann als therapeutische Maßnahme punktiert. Die dabei entnommene Synovialflüssigkeit wird analysiert um Anhaltspunkte für das lokale Geschehen zu bekommen, und um Aufschluss über die hier vorliegende Erkrankung zu erhalten. Untersucht werden physikalische und chemische Eigenschaften, Entzündungsmarker (C-reaktives Protein; das Vorkommen von Leukocyten), sowie eventuell das Vorkommen von Kristallen (Gicht) oder Antikörpern (z. B. Rheumafaktor). Punktiert wird bei sehr vielen entzündlichen, aber auch nicht entzündlichen Erkrankungen.

Häufig ist zu Beginn einer Arthritis nicht erkennbar, um welche Art von Arthritis es sich handelt (undifferenzierte Arthritis). Es gibt viele verschiedene Formen, von denen die RA nur eine ist. Eine gute Prognose des zukünftigen Verlaufs ist wünschenswert, um schwerwiegende Folgen verhindern zu können.

Zur Zeit werden für die Diagnose einer RA die sogenannten ACR-Kriterien verwendet, wobei mindestens 4 von möglichen 7 Kriterien erfüllt sein müssen, um die Erkrankung als RA zu klassifizieren. Dies ist zu Beginn der Krankheit jedoch häufig noch nicht möglich. Noch schwieriger ist es, früh eine Prognose über den weiteren Verlauf der RA zu machen, was für die Wahl der Therapie große Bedeutung hat. Unter den 7 ACR-Kriterien befindet sich nur ein Laborparameter (Rheumafaktor: anti-IgG). In jüngerer Zeit gibt es einen zweiten Antikörper (anti-CCP, anti-citrulliniertes-Fibrinogen u. a. citrullinierte Proteine bzw. Peptide), der ebenfalls für die Diagnose einer RA zunehmend an Bedeutung gewinnt. Beide Autoantikörper überlappen weitgehend, jedoch gibt es auch RA-Patienten, die nur einen von beiden oder gar keinen entwickeln. Auch kommen beide Autoantikörper in gewissem Maße bei anderen Patienten und Gesunden vor. Obwohl insbesondere anti-CCP-Antikörper recht spezifisch sind und relativ früh auftreten, sind sie als Reaktion auf zuvor eingetretene Veränderungen des Individuums zu sehen. Es ist daher wünschenswert, weitere Parameter oder Marker zu finden, die direkt mit diesen frühen Veränderungen einhergehen und eine möglichst frühzeitige und verlässliche Diagnose und Prognose ermöglichen.

Wie viele andere Krankheiten des rheumatischen Formenkreises ist die RA eine Krankheit, die in vielen verschiedenen Formen auftritt. Gegenwärtig werden hauptsächlich eine seropositive und eine seronegative Form unterschieden, und zwar aufgrund der An- oder Abwesenheit von Rheumafaktor. Man beginnt auch, eine anti-CCP-positive und eine anti-CCP-negative Form zu definieren. Möglicherweise werden weitere Klassifikationen möglich, wenn weitere Marker eingesetzt werden können, und auch Kombinationen von mehreren Markern möglich werden. Genetische Marker werden zurzeit gar nicht verwendet.

Auch zur Verlaufskontrolle und zur Abschätzung des individuellen Risikos sind neue Marker wünschenswert, insbesondere auch solche, die sich sehr früh finden lassen, also möglichst noch vor dem Auftreten von Antikörpern.

Da Punktate ausschließlich aus klinischen Gründen entnommen werden, kann die Synovialflüssigkeit als "Abfallprodukt" angesehen werden, das in diagnostischen Verfahren vorteilhaft eingesetzt werden kann.

Gegenstand der vorliegenden Erfindung ist daher ein in vitro Verfahren zur Klassifikation und Prognose einer rheumatisch-entzündlichen Erkrankung, bei dem die Gegenwart oder Abwesenheit des Proteins Hdj2 in Synovialflüssigkeit bestimmt wird.

Mit dem Verfahren kann in einem frühen Stadium die rheumatoide Arthritis unterschieden werden von anderen Erkrankungen des rheumatischen Formenkreises wie: reaktiver Arthritis und Psoriasis-Arthritis.

Darüber hinaus können milde Verlaufsformen einer entzündlichen Polyarthritis von einer rheumatoiden Arthritis unterschieden werden.

Bei den erfindungsgemäßen Verfahren handelt es sich in bevorzugter Ausführungsform um immunologische Verfahren. Dabei wird das Protein Hdj2 in Synovialflüssigkeit, die von Patienten erhalten wurde, die eine frühe Form einer Erkrankung des rheumatischen Formenkreises zeigen, immunologisch untersucht.

Der Nachweis der Proteins Hdj2 in der Synovialflüssigkeit wird bevorzugt mit einem Antikörper durchgeführt, wobei grundsätzlich polyklonale und monoklonale Antikörper eingesetzt werden können. Da die Aminosäuresequenz bekannt ist (SEQ ID NO:2), ist es ohne weiteres möglich, Antikörper hiergegen herzustellen. Dabei können beispielsweise Kaninchen, Mäuse oder Ratten mit dem humanen Protein Hdj2, gegebenenfalls unter Zugabe von Adjuvantien, immunisiert werden. Antiserum dieser Tiere kann, vorzugsweise in gereinigter Form, zur Detektion von Hdj2 verwendet werden. Die Milzzellen der immunisierten Tiere können alternativ mit Tumorzellen fusioniert und die immortalisierten, monoklonale Antikörper produzierenden Zellinien vereinzelt und propagiert werden. Nach Charakterisierung der Epitope, an die die jeweiligen monoklonalen Antikörper binden können, werden diejenigen monoklonalen Antikörper ausgewählt, die in den immunologischen Tests die besten Ergebnisse liefern. Alternativ hierzu können auch käuflich erhältliche monoklonale und polyklonale Antikörper eingesetzt werden.

Bei der immunologischen Charakterisierung der Synovialflüssigkeit kann so vorgegangen werden, dass die Proteine der Synovialflüssigkeit auf einem Elektrophorese-Gel (SDS-PAGE) aufgetrennt werden und die Proteine dann auf eine geeignete Membran (Nitrozellulose oder Nylon) übertragen werden. Diese Membranen werden dann mit den gegen Hdj2 gerichteten Antikörpern umgesetzt und die gebundenen Antikörper werden mit Anti-Antikörpern nachgewiesen, wobei die Anti-Antikörper in der Regel gegen den Fc-Teil der anti-Hdj2-spezifischen Antikörper gerichtet sind. Die Anti-Antikörper sind üblicherweise mit einem farberzeugenden oder lichterzeugenden Enzym verbunden, das eine farbgebende Reaktion katalysiert oder einen Film schwärzt.

Alternativ hierzu kann der immunologische Nachweis mit ELISA-Tests durchgeführt werden. Hierbei können beispielsweise geeignete Antikörper, bevorzugt monoklonale Antikörper, an die Wand der Mikrotiterplatten geheftet werden. Anschließend wird Synovialflüssigkeit zugegeben und gewaschen. Falls das Protein Hdj2 vorhanden ist, bindet dieses dann an die ELISA-Platten. Der Nachweis des Proteins Hdj2 wird dann mit Antikörpern bewirkt, die gegen bestimmte Epitope des Hdj2 gerichtet sind und die mit einer farbgebenden Komponente verbunden sind.

Das erfindungsgemäße Verfahren kann mit geeigneten Testkits durchgeführt werden. Derartige Testkits beinhalten als Bestandteil ein immunologisches Reagens, das den spezifischen Nachweis des Proteins Hdj2 in Synovialflüssigkeit ermöglicht. In bevorzugter Ausführungsform handelt es sich hierbei um monoklonale Antikörper, die spezifisch und mit ausreichender Sensitivität an geeignete Epitope des Proteins Hdj2 binden.

Darüber hinaus weisen Testkits übliche Hilfsmittel und Hilfsstoffe auf, die zu der Durchführung eines erfindungsgemäßen Verfahrens erforderlich sind. Es kann sich hierbei um vorbeschichtete Mikrotiterplatten oder um Membranen handeln sowie die zu der Durchführung des Testverfahrens erforderlichen Reagenzien.

In einer besonders bevorzugten Ausführungsform der vorliegenden Erfindung werden gegen das Protein Hdj2 gerichtete Antikörper zur Differentialdiagnose einer entzündlich-rheumatischen Erkrankung in einer Frühphase der Erkrankung eingesetzt.

Der Ausdruck "Antikörper" oder "monoklonaler Antikörper", wie in der vorliegenden Anmeldung verwendet, ist so zu verstehen, dass er gleichzusetzen ist mit den entsprechenden bindenden Teilen eines Antikörpers. In der Antikörpertechnologie hat es sich als zweckmäßig erwiesen, anstelle der kompletten Antikörper auch monospezifische Antikörperfragmente einzusetzen, die an die gewünschten Epitope binden. Der Ausdruck "Antikörper" kann in der vorliegenden Anmeldung ersetzt werden durch Fab-Fragmente, Fv-Fragmente, Single Chain Antikörper-Fragmente (scFv), Disulfidbrücken-stabilisierte Fv-Fragmente, V_{H}- und Kamel-Antikörper; multivalente Antikörperfragmente, bifunktionelle Antikörper und bispezifische Antikörper.

Proben von SF wurden mittels SDS-PAGE aufgetrennt, und im Immunoblot mit spezifischen Antikörpern gefärbt. Erfindungsgemäß wurden Antikörper gegen Hdj2 (KA2A5.6) eingesetzt. Bei diesem zeigten sich häufig Banden mit dem erwarteten Molekulargewicht.

Es wurden auch weitere immunhistochemische Analysen von Synovialgewebe (Gefrierschnitte), von RA-Patienten sowie von Kontrollpersonen mit verschiedenen Formen von Osteoarthrose (OA), darunter ein Karpaltunnelsyndrom gemacht. Dabei wurde anti-Hdj2 und Antikörper, mit deren Hilfe die Hdj2-exprimierenden Zellen charakterisiert werden konnten, eingesetzt.

Die Spezifität des Antikörpers wurde untersucht. Es zeigte sich, dass der Antikörper nur Zellen anfärbt, die auch Transkripte des Hdj2 (mRNA des Gens DNAJA1) enthalten. Es zeigte sich, dass in Synovialgewebe von RA Patienten Transkripte des Hdj2 zu finden sind.

Die genauen Diagnosen und alle sonstigen Unterlagen der Patienten wurden zusammengestellt. Zunächst wurden die Patienten in zwei Klassen eingeordnet, nämlich die mit einer gesicherten RA (Diagnose gegeben, >4 ACR-Kriterien waren nachweisbar vorhanden), und die mit einer gesicherten anderen Diagnose (z.B. reaktive Arthritis, Psoriaris Arthritis usw.). Von insgesamt 130 Patienten hatten 50 eine RA und 80 keine RA.

Überraschenderweise wurde Hdj2 in vielen Punktaten gefunden.

Danach wurden die Diagnosen geprüft und weitere SF untersucht. Es ergab sich im Verlauf der Untersuchung eine signifikante Korrelation zwischen dem Auftreten von Hdj2 in SF und der Diagnose RA (p = 0.021). Auch konnte gezeigt werden, dass Hdj2 immunhistochemisch nur in Schnitten von Synovialgeweben von Patienten mit RA, nicht aber bei OA, überexprimiert war.

In den SF konnte semiquantitativ die Menge an Hdj2 klassifiziert werden (0, 1, 2, 3, 4). Mit dieser Klassifikation und dem Vorkommen als solchem wurde versucht, Beziehungen zu anderen Parametern zu finden. Es gab bei RA Patienten eine positive Korrelation mit dem Entzündungsgrad, bestimmt nach der Anzahl der das Gelenk infiltrierenden Leukocyten. In vielen SF war bereits zuvor im Routinelabor der Rheumafaktor bestimmt worden. Es wurden nun zusätzlich die anti-CCP-Antikörper in allen SF bestimmt. Bei beiden Autoantikörpern ergaben sich Korrelationen mit der Diagnose RA (jeweils mit p<0.001). Auch wurden Korrelationen zwischen der Präsenz von Hdj2 und beiden Autoantikörpern (Rheumafaktor: p = 0.038 bzw. anti-CCP: p = 0.016) gefunden. Jedoch gab es keine komplette Überlappung. Deshalb erscheint Hdj2 in der SF als zusätzlicher Marker geeignet, z.B. für Subgruppenbildungen.

Wichtig erschien auch die Frage, ob extrazelluläres Hdj2 bei RA Patienten auch im Blut zu finden ist. Es wurden deshalb 14 Plasmen von RA Patienten getestet, die zum gleichen Zeitpunkt relativ viel Hdj2 in der SF enthielten. In diesen Immunoblots konnte kein Hdj2 in den Plasmen nachgewiesen werden.

Hdj2 in der Synovialflüssigkeit ist ein früher Marker einer RA. Das Ergebnis eines Gewebes, das von einer Patientin mit einem Karpaltunnel-Syndrom stammte, und das als Kontrolle für keine RA diente, war zunächst unverständlich. Jedoch konnte bei den Recherchen festgestellt werden, dass diese Patientin mehr als ein Jahr später eine RA entwickelte. Es gibt Hinweise darauf, dass Patienten mit Karpaltunnel-Syndrom relativ oft eine RA entwickeln. Die Überexpression von Hdj2 ist also ein frühes Zeichen für eine beginnende - und noch nicht diagnostizierte - RA.

Daraufhin wurde auch SF von Patienten mit unklarer Diagnose analysiert. In mehreren Fällen, bei denen Hdj2 bereits in SF nachweisbar war, wurde RA identifiziert, bevor die Diagnose RA nach den gültigen Kriterien gestellt werden konnte. Hdj2 eignet sich daher als Marker für eine Früherkennung von RA, und auch als Marker für eine Differentialdiagnose.

Durch die nachfolgenden Beispiele wird die Erfindung verdeutlicht.

### Beispiel 1

Eine 53-jährige Patientin klagt über Polyarthralgien der Wirbelsäule und Beschwerden im Bereich der Handgelenke. Sie wird internistisch, neurologisch und rheumatologisch untersucht. Es findet sich aktuell klinisch und auch serologisch kein Hinweis auf ein entzündliches Gelenkgeschehen (auch kein Rheumafaktor). Ausdrücklich wird erwähnt, dass die Patientin zwar HLA-B27 positiv ist, aber keinen M.Bechterew hat. Die Patientin ist durch eine schwerst-behinderte Tochter körperlich deutlich gefordert. Orthopädisch erhält sie die Diagnose eines leichten sekundären Karpaltunnelsyndroms, ausgehend von der Wirbelsäule.

Das Karpaltunnelsyndrom wird 7 Monate später operiert. Dabei entnommenes Synovialgewebe wird im Gefrierschnitt bezüglich der Expression von Hdj2 (und Hsc70) untersucht. Es stellt sich heraus, dass beide Moleküle bei dieser Patientin stark erhöht nachgewiesen werden können, was zuvor nur bei Patienten mit einer etablierten RA gelang, während Kontrollpersonen mit verschiedenen anderen Diagnosen (OA) negativ waren.

Ein Jahr später stellt die Patientin sich bei einem anderen Rheumatologen vor, mit Verdacht auf eine chronische Polyarthritis. Diese Diagnose war 2 Monate zuvor während einer Kur zum ersten Mal gestellt worden. Es wurde festgestellt, dass eine Morgensteifigkeit für 3 Stunden, ein Befall der Fingergelenke, symmetrisch, sowie positive Röntgenzeichen, bestehen. Die Diagnose lautete nun seronegative, ANA positive, rheumatoide Arthritis (mit ≥ ACR-Kriterien erfüllt).

Dieses Beispiel zeigt die Möglichkeit der Früherkennung einer RA bei einer Patientin mit Symptomen einer entzündlich-rheumatischen Erkrankung.

### Beispiel 2

Von insgesamt sechs Patienten, bei denen sich erst deutlich nach Auftreten von Symptomen einer rheumatischen Erkrankung die Diagnose rheumatoide Arthritis bestätigte, wurde Synovialflüssigkeit entnommen.

Die Synovialflüssigkeit wurde mit Hilfe einer SDS-PAGE Elektrophorese aufgetrennt und die Proteinbanden wurden auf Nitrozellulose geblottet. Nach Blockierung unspezifischer Bindungsstellen wurde mit einem primären Antikörper gegen Hdj2 über Nacht bei 4°C im Puffer reagieren gelassen. Anschließend wurde dreimal gewaschen und der Anti-Hdj2-Antikörper wurde mit Hilfe eines Anti-Maus-Antikörpers, der aus der Ziege gewonnen wurde, nachgewiesen. Anschließend wurde der Film entwickelt und die Bindung wurde semiquantitativ nachgewiesen. Die Ergebnisse sind in der Figur 1 zusammengestellt, die Tabelle 1 beinhaltet.

### Beispiel 3

Um den zeitlichen Verlauf des Auftretens von Hdj2 in der Synovialflüssigkeit zu dokumentieren, wurde Hdj2 in der Synovialflüssigkeit von Patienten bestimmt, die erst seit wenigen Wochen bis Monaten Symptome hatten, zum ersten Mal punktiert wurden, und kurz darauf erstmalig eine sichere Diagnose erhielten. Hierbei wurde Hdj2 nachgewiesen bei Patienten, die bereits zu diesem frühen Zeitpunkt ≥4 ACR Kriterien erfüllten. Bei Patienten mit eindeutigen anderen Diagnosen wurde Hdj2 nicht nachgewiesen. (Patienten mit undifferenzierter Arthritis oder unklarer Diagnose zu diesem Zeitpunkt wurden in diese Untersuchung nicht einbezogen). Die Messungen wurden in einem späteren Stadium der Erkrankung durchgeführt, und zwar in einem Zeitraum zwischen etwa 0,5 und 1 Jahr nach Auftreten der ersten Symptome (bzw. der Erstdiagnose). Die erhaltenen Daten sind in Figur 2 aufgeführt, die die Tabelle 2 zeigt.

### Beispiel 4

Das Vorhandensein des Hdj2-Proteins in der Synovialflüssigkeit wurde auch zu späteren Zeitpunkten nach dem Auftreten der ersten Symptome gemessen. In Figur 3 sind die Daten von Patienten in der Tabelle 3 zusammengestellt, bei denen die Messung von Hdj2 in der Synovialflüssigkeit in den ersten beiden Jahren nach Auftreten der Symptome bestimmt wurde. Die Bestimmung der Hdj2-Werte wurde also in einem Zeitraum bis zu 2 Jahren nach Auftreten der ersten Symptome (bzw. der Erstdiagnose) durchgeführt. Die Tabelle 3 belegt, dass sämtliche Patienten, die die Diagnose rheumatoide Arthritis (RA) erhielten, auch Hdj2-Protein in der Synovialflüssigkeit aufwiesen. Patienten, die an anderen Erkrankungen des rheumatoiden Formenkreises litten, zeigten kein Hdj2-Protein.

## Patentansprüche

1. In vitro Verfahren zur Bestimmung des Typs einer entzündlich-rheumatischen Erkrankung, nämlich zur Unterscheidung der rheumatoiden Arthritis von reaktiver Arthritis und Psoriasis-Arthritis. **dadurch gekennzeichnet, dass** die Gegenwart oder Abwesenheit des Proteins Hdj2 in Synovialflüssigkeit immunologisch in einem frühen Stadium der Erkrankung, nämlich in einer Zeitspanne von bis zu zwei Jahren seit dem Auftreten der ersten Symptome einer entzündtich-rheumatischen Erkrankung, bestimmt wird.

2. Verfahren nach Anspruch 1, **dadurch gekennzeichnet, dass** bei dem immunologischen Nachweisverfahren wenigstens ein Antikörper eingesetzt wird, der spezifisch an ein Epitop des Proteins Hdj2 bindet.

3. Verfahren nach einem der Ansprüche 1 oder 2, **dadurch gekennzeichnet, dass** es ein ELISA-Verfahren ist.

4. Verfahren nach einem der Ansprüche 1 oder 2, **dadurch gekennzeichnet, dass** das Verfahren ein Western Blot-Verfahren ist.

5. Verwendung von monoklonalen Antikörpern gegen das Protein Hdj2 zur In vitro - Differentialdiagnose einer entzandllch-rheumatlschen Erkrankung im Frühstadium einer entzündtich-rheumatischen Erkrankung, nämlich In einer Zeitspanne von bis zu zwei Jahren seit dem Auftreten der ersten Symptome einer entzündlich-rheumatischen Erkrankung, nämlich zur Unterscheidung der rheumatoiden Arthritis von reaktiver Arthritis und Psorlasis-Arthritis.

## Claims

1. In vitro method for determining the type of inflammatory rheumatic disease, namely for the differentiation of rheumatoid arthritis from reactive arthritis and psoriasis-arthritis, **characterized in that** the presence or absence of the protein Hdj2 in synovial fluid is determined immunologically at an early stage of the disease, namely in a time span of up to two years from the appearance of the first symptoms of an inflammatory rheumatic disease.

2. The method as claimed in claim 1, **characterized in that** the immunological detection method makes use of at least an antibody which binds specifically to an epitope of the protein Hdj2.

3. The method as claimed in either claim 1 or 2, **characterized in that** it is an ELISA method.

4. The method as claimed in either claim 1 or 2, **characterized in that** the method is a Western blot method.

5. The use of monoclonal antibodies against the protein Hdj2 for the in vitro differential diagnosis of an inflammatory rheumatic disease, namely for the differentiation of rheumatoid arthritis from reactive arthritis and psoriasis-arthritis, at the early stage of an inflammatory rheumatic disease, namely in a time span of up to two years from the appearance of the first symptoms of an inflammatory rheumatic disease.

## Revendications

1. Procédé in vitro de détermination du type d'une affection rhumatismale inflammatoire, à savoir pour pouvoir distinguer l'arthrite rhumatoïde de l'arthrite réactive et de l'arthrite psoriasique, **caractérisé en ce que** la présence ou l'absence de la protéine Hdj2 dans la synovie est déterminée de façon immunologique à un stade précoce de l'affection, à savoir dans un laps de temps allant jusqu'à deux ans dès l'apparition des premiers symptômes d'une affection rhumatismale inflammatoire.

2. Procédé selon la revendication 1, **caractérisé en ce qu'**au cours du dépistage immunologique, au moins un anticorps se liant spécifiquement à un épitope de la protéine Hdj2 est utilisé.

3. Procédé selon l'une quelconque des revendications 1 ou 2, **caractérisé en ce qu'**il s'agit d'un procédé ) ELISA.

4. Procédé selon l'une quelconque des revendications 1 ou 2, **caractérisé en ce que** le procédé est un procédé Western-Blot.

5. Utilisation d'anticorps monoclonaux contre la protéine Hdj2 pour le diagnostic différentiel in vitro d'une affection rhumatismale inflammatoire au stade précoce d'une affection rhumatismale inflammatoire, à savoir dans un laps de temps allant jusqu'à deux ans dès l'apparition des premiers symptômes d'une affection rhumatismale inflammatoire, à savoir pour pouvoir distinguer l'arthrite rhumatoïde de l'arthrite réactive et de l'arthrite psoriasique.
